(19) Europäisches Patentamt
European Patent Office
Office européen des brevets

(11) Publication number: **0 109 231**
**B1**

(12) # EUROPEAN PATENT SPECIFICATION

(45) Date of publication of patent specification: **22.10.86**

(21) Application number: **83306690.5**

(22) Date of filing: **03.11.83**

(51) Int. Cl.$^4$: **C 07 D 491/107,**
C 07 C 87/40, C 07 D 453/02 //
C07D311/22, A61K31/415
,(C07D491/107, 311:00,
235:00)

(54) **Sorbinil by optical resolution with aminopinane derivatives.**

(30) Priority: **10.11.82 US 440686**

(43) Date of publication of application:
**23.05.84 Bulletin 84/21**

(45) Publication of the grant of the patent:
**22.10.86 Bulletin 86/43**

(84) Designated Contracting States:
**AT BE CH DE FR GB IT LI LU NL SE**

(56) References cited:
**US-A-4 177 282**

**S.H. WILEN, Resolving Agents and Resolutions in Organic Chemistry, Wiley, 1971, page 108**

(73) Proprietor: **PFIZER INC.**
**235 East 42nd Street**
**New York, N.Y. 10017 (US)**

(72) Inventor: **Sysko, Robert J.**
**6 Stone Cliffe Drive**
**Niantic Connecticut 06357 (US)**

(74) Representative: **Graham, Philip Colin Christison et al**
**Pfizer Limited Ramsgate Road**
**Sandwich, Kent CT13 9NJ (GB)**

**EP 0 109 231 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Background of the Invention

S-6-Fluorospiro[chroman-4,4'-imidazolidine]-2',5'-dione, also named S-2,3-dihydro-6-fluorospiro[4H-1-benzopyran-4,4'-imidazolidine]-2',5'-dione (USAN: sorbinil) of the formula

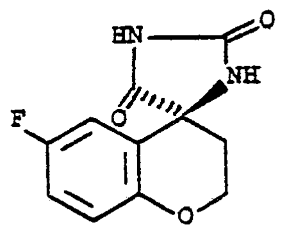

---(I)

is a highly potent aldose reductase inhibitor having especial value in effectively controlling the chronic complications of diabetes mellitus. (Sarges, U.S. Patent No. 4,130,714). The present invention concerns an improved process for sorbinil and intermediates used in that process.

Heretofore, sorbinil was prepared by resolution of racemic 2,3-dihydro-6-fluorospiro[4H-1-benzopyran-4,4'-imidazolidine]-2',5'-dione by using high solvent volumes and highly toxic brucine as the resolving agent (U.S. Patent No. 4,130,714).

Surprisingly, we have found that resolution of the racemate is accomplished by crystallization from solution in lower alkanols either (a) directly via the (−)-3-aminomethylpinane salt of sorbinil; or (b) by a double resolving agent technique, via a mother liquor concentrate of either the (+)-3-aminomethylpinane or the (−)-2-amino-2-norpinane salt followed by the quinine salt. Good yields of sorbinil are obtained with much lower solvent volumes, and at the same time, the use of a highly toxic resolving agent is avoided. The efficiency of this process is further enhanced by isolating the undesired enantiomer from mother liquors and recycling to fresh racemate, via precursor 6-fluoro-4-chromanone.

Sorbinil has also been more recently prepared by an alternative synthesis in which the required chirality is induced during the synthetic sequence (Sarges, U.S. Patent No. 4,286,098).

Summary of the Invention

The present invention encompasses a process for preparing sorbinil, or a pharmaceutically acceptable cationic salt thereof, which comprises the steps of:

(a) crystallizing the (−)-3-aminomethylpinane salt of sorbinil from a solution of racemic hydantoin of the formula

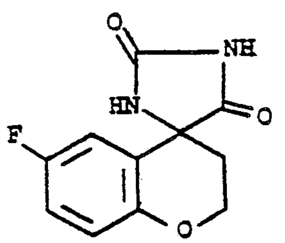

---(II)

in a solvent consisting of a lower alkanol or a mixture of lower alkanols, and

(b) converting said aminopinane salt to said sorbinil or pharmaceutically acceptable salt thereof.

The present invention also encompasses a process for preparing sorbinil, or a pharmaceutically acceptable cationic salt thereof, which comprises the steps of:

(a) crystallizing either the (+)-3-aminomethylpinane or the (−)-2-amino-2-norpinane salt of the enantiomer of sorbinil from a solution of racemic hydantoin of the formula (II) in a solvent consisting of a lower alkanol or a mixture of lower alkanols;

(b) isolating a concentrate of sorbinil from the mother liquor thereof;

(c) crystallizing the quinine salt of sorbinil from a solution of the said sorbinil concentrate; and

(d) converting said quinine salt of sorbinil to said sorbinil or pharmaceutically acceptable salt thereof.

The present invention further encompasses intermediate chiral amine salts of sorbinil, wherein said chiral amine is selected from the group consisting of (−)-3-aminomethylpinane, (+)-3-aminomethylpinane, (−)-2-amino-2-norpinane and quinane. Particularly valuable are those amine salts isolated in crystalline form, viz., the (−)-3-aminomethylpinane salt and the quinine salt of sorbinil.

3-Aminomethylpinane and 2-amino-2-norpinane are, respectively, of the formulas:

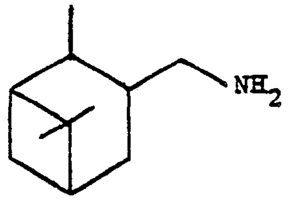

and

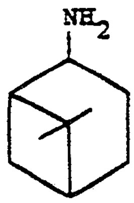

.

Detailed Description of the Invention

The processes of the present invention are readily carried out. To prepare sorbinil by direct resolution, the racemate is slurried or dissolved in the lower alkanol or a mixture of lower alkanols, by heating to 50—90°C. The preferred solvent is a mixture of methanol and isopropanol, in a ratio of about 1:1 by volume, conveniently heated to reflux (about 69—71°C). Substantially one molar equivalent of (−)-3-aminomethylpinane, conveniently dissolved in a portion of the solvent, is added to the hot slurry or solution to form a solution of diastereomeric salts. The amine salt solution is cooled slowly to about 0—5°C and the resulting crystalline solids usually granulated for a period of time prior to isolation. Such slow cooling and granulation minimizes occlusion of the undesired diastereomeric salt. The volume of solvent is selected so as to maximize recovery of sorbinil salt, while minimizing the amount of the undesired enantiomeric salt which crystallizes. If the product contains any significant amount of the latter, it is readily removed by optional recrystallization from the same alkanol or mixed alkanol solvent. The (−)-3-aminomethylpinane salt of sorbinil is then converted to sorbinil or a pharmaceutically acceptable salt thereof by standard methods well known in the art. For example, the amine salt is distributed between at least one equivalent of aqueous acid and a water immiscible organic solvent, and sorbinil recovered by separation and concentration of the organic layer and/or the addition of a non-solvent. If a pharmaceutically acceptable cationic salt is desired, the sorbinil is extracted back into water containing substantially one equivalent of the cation in base form (e.g., NaOH, KOH, $NH_4OH$) and the salt isolated from the aqueous layer by concentration or freeze drying.

To prepare sorbinil by the so-called double resolving agent technique, the initial stage of the process is fully analogous to the direct process, except that the *enantiomer* of sorbinil is crystallized directly as either the (+)-3-aminomethylpinane or the (−)-2-amino-2-norpinane salt, providing a mother liquor concentrate, respectively, of either the (+)-3-aminomethylpinane or the (−)-2-amino-2-norpinane salt of sorbinil. Either of these concentrates is then converted by standard methods (such as detailed above) to a partially resolved concentrate of sorbinil. Sufficient resolution is now attained so as to be readily completed by crystallization of sorbinil as its quinine salt. The conditions employed are essentially as detailed above, but quinine is now substituted for the aminopinane derivative, with about 4:1 isopropanol:methanol now the preferred alkanol solvent. The quinine salt is also optionally recrystallized as detailed above and finally converted to sorbinil, by the standard methods detailed above.

The racemic 6-fluorospiro[4H-1-benzopyran-4,4'-imidazoline]-2',5'-dione required for the present sorbinil processes is derived from 6-fluoro-4-chromanone according to the method of Sarges, U.S. Patent No. 4,130,714. The efficiency of the overall process is greatly increased by recovering the enantiomer of sorbinil (usually also containing racemate) from mother liquors. The latter is recycled via 6-fluoro-4-chromanone to produce additional racemate suitable for optical resolution to sorbinil. The 6-fluoro-4-chromanone for recycling is regenerated according to the method of European patent application publication No. 0111387 as also disclosed in specific Examples below.

The present invention is illustrated by the following examples. However, it should be understood that the invention is not limited to the specific details of these examples.

### Example 1

Sorbinil via its (−)-3-Aminomethylpinane Salt

(−)-3-Aminomethylpinane HCl, 9.5 g (46.6 mmole), ethyl acetate (186 ml) and 1N NaOH (93 ml) were stirred vigorously for 10 minutes. The organic layer was separated, washed 1 × 93 ml $H_2O$, dried ($MgSO_4$) and stripped to yield (−)-3-aminomethylpinane free base as an oil, 7.75 g (99%) $[alpha]_D^{25} = -54.85°$ (c = 1 in methanol), which was then dissolved in 20 ml methanol.

Racemic 6-fluorospiro[4H-1-benzopyran-4,4'-imidazolidine]-2',5'-dione (10.0 g, 42.3 mmoles) was dissolved in 214 ml 2-propanol and 194 ml methanol, heated to reflux, and the above solution of amine added, providing a clear solution at the 71° reflux temperature. The solution was slowly cooled (crystallization began at 27°) and stirred at room temperature for 6 hours. The crude (−)-3-aminomethylpinane salt of sorbinil was recovered by filtration and dried *in vacuo* at 40°, 6.81 g., m.p. 127.5—196° (dec), $[alpha]_D^{25} = -8.2°$ (c = 1 in methanol). Step yield: 79.6%. The mother liquor therefrom is evaporated to dryness to yield crude (−)-3-aminomethylpinane salt of the enantiomer of sorbinil, the main contaminant being the (−)-3-aminomethylpinane salt of sorbinil.

Crude sorbinil salt (6.7 g) was dissolved at reflux in 80.4 ml of 1:1 methanol:isopropanol, cooled slowly to room temperature and the resulting solids granulated 2 hours. Purified salt was recovered by filtration

with 4 ml 1:1 methanol:isopropanol wash, and dried *in vacuo* at 40°C., 4.42 g, m.p. 119—208° (dec.), $[alpha]_D^{25}$ = +3.9° (c = 1 in methanol). Step yield: 66%.

Purified salt (4.33 g, 10.7 mmole) was distributed into 107.5 ml ethyl acetate and 53.8 ml 1N HCl. The organic layer was separated, washed 1 × 54 ml 1N HCl, 1 × 54 ml $H_2O$ and 1 × 54 ml brine, dried ($MgSO_4$), filtered, evaporated *in vacuo* to a slurry, chased 3 × 50 ml ethyl acetate to a final volume of 12 ml, granulated 3 hours, filtered and dried *in vacuo* at 40° to yield sorbinil, 2.18 g, mp 234—242°, $[alpha]_D^{25}$ = +51.2° (c = 1, methanol). Step yield: 86.2%. Alternatively, the dried organic layer is extracted with an equivalent of 1N NaOH. The extract is separated and freeze dried to yield the sodium salt of sorbinil.

The above aqueous layers from sorbinil recovery were combined (226 ml), combined with 113 ml $CH_2Cl_2$ and the pH adjusted to 10.0 with 25% NaOH. The aqueous layer was separated and extracted with 55 ml fresh $CH_2Cl_2$. The $CH_2Cl_2$ layers were combined, evaporated *in vacuo* to 60 ml, washed 1 × 30 ml $H_2O$, dried ($MgSO_4$) and evaporated to yield recovered (−)-3-aminomethylpinane; oil, 1.58 g, $[alpha]_D^{25}$ = −55.5° (c = 1, methanol).

Sorbinil was further purified by dissolving in 20 ml hot ethanol, concentrating to $\frac{1}{2}$ volume and granulating 4 hours at room temperature. Purified sorbinil was recovered by filtration and dried at 40° *in vacuo*, 1.82 g, mp 234—241.5°, $[alpha]_D^{25}$ = +53.1. Step yield: 91%. Overall yield: 41.2%.

By the same extraction techniques as those described above, the above crude (−)-3-aminomethylpinane salt of the enantiomer of sorbinil is converted to (−)-3-aminomethylpinane, suitable for recycle, and to the enantiomer of sorbinil, contaminated with racemate.

Example 2

Crude 6-Fluoro-4-Chromanone from Sorbinil Enantiomer and Racemate

Levorotatory (R-) and/or racemic (RS-) 6-fluorospiro-[chroman-4,4'-imidazolidine]-2',5'-dione (100 g, 0.423 mmole) was slurried in 750 ml $H_2O$. $Ba(OH)_2.8H_2O$ (267.0 g, 0.846 mole) was added and the resulting thin slurry refluxed 48 hours. The resulting heavy suspension was cooled to 60-65° and $NH_4CO_3$ (100 g, 0.876 mole) added. The slurry was then stirred 30 minutes and filtered at 50-55° with 300 ml of warm water wash of the collected inorganic salts. The combined filtrate and wash was adjusted from pH 8.5 to 4.5—5.0 with hydrochloric acid. To the acidified solution, N-chlorosuccinimide (57.0 g, 0.427 mole) was added portionwise over 5 hours at 30-45 minute intervals. The resulting slurry was stirred 17 hours at room temperature, then 1 hour at 15°. Solids were recovered by filtration, taken up in $CH_2Cl_2$ treated with activated carbon, and $CH_2Cl_2$ displaced with hexane to a pot temperature of 68—69° and a final volume of 400—500 ml, during which crystallization occurred. After cooling and digestion for 1 hour at 20—25°, purified title product was recovered by filtration, 50.2 g, having the physical properties of the known material.

Title product prepared in this manner contains 6-fluoro-4-chloriminochroman as an impurity. The latter interferes with further use of title product in the synthesis of additional sorbinil. Said impurity is removed (being converted to the desired 6-fluoro-4-chromanone) according to the following Example.

Example 3

Purification of Crude 6-Fluoro-4-chromanone by Hydrogenation

Crude 6-fluoro-4-chromanone, containing 6-fluoro-4-chloriminochroman as an impurity (5.0 g), 5% Pd/C (50% water wet, 0.25 g), and 1:1 $H_2O$:$C_2H_5OH$ (100 ml) were combined and the mixture hydrogenated at 45 psig of hydrogen (4 bars) for 2 hours, by which time tlc on silica gel (using toluene:methyl ethyl ketone:acetic acid 5:2:1 as eluant) indicated absence of faster moving chlorimine ($R_f$ 0.8) in the 6-fluoro-4-chromanone ($R_f$ 0.7). The reaction mixture was diluted with 100 ml of methanol (to completely dissolve solids other than catalyst), the catalyst recovered by vacuum filtration on a pad of diatomaceous earth, and the filtrate evaporated *in vacuo* to 50 ml (from a water bath at 45°), cooled to 5°, granulated for 15 minutes and filtered to yield purified title product, 2.65 g, mp 108—112°, tlc as indicated above.

Example 4

Sorbinil via Its (−)-2-Amino-2-norpinane and Quinine Salts

Racemic 6-fluorospiro[4H-1-benzopyran-4,4'-imidazolidine]-2',5'-dione (25 g, 0.106 mole) was slurried in 250 ml isopropanol and 100 ml methanol and heated to reflux. A solution of (−)-2-amino-2-norpinane (14.73 g, 0.106 mole) in 150 ml methanol was added to the hot slurry over 16 minutes. Complete dissolution occurred when about 75 ml of the amine solution had been added. The solution was held briefly at reflux and then cooled slowly to 0—5°C. The resulting slurry was granulated 2 hours and (−)-2-amino-2-norpinane salt of sorbinil enantiomer recovered by filtration (with 25 ml of 1:1 isopropanol:methanol wash) and dried at 40°C *in vacuo*, 18.71 g, m.p. 170—187°C, $[alpha]_D^{25}$ = −24.3° (c = 1, methanol). Yield 94.5% of theory.

The combined mother liquor and wash (515 ml), containing primarily the (−)-2-amino-2-norpinane salt of sorbinil, was concentrated *in vacuo* to 250 ml and diluted with 512 ml of ethyl acetate and 256 ml 1N HCl. The organic layer was separated, washed 1 × 256 ml fresh 1N HCl, 1 × 256 ml $H_2O$ and 1 × 256 ml brine, dried ($MgSO_4$), evaporated to 100 ml *in vacuo* and the remaining ethyl acetate chased with 3 × 100 ml of hexane to a final volume of 75 ml. The resulting slurry was granulated for 2 hours and filtered to yield crude sorbinil, dried *in vacuo* at 40°C, 11.56 g, m.p. 225—237°C, $[alpha]_D^{25}$ = +28.5°. Step yield: 92.5%.

Crude sorbinil (11.44 g, 48.4 mmole) was slurried in 137 ml isopropanol and heated to reflux. A solution of quinine trihydrate (18.3 g, 48.4 mmole) in 34.3 ml methanol was added over 7 minutes, producing a clear solution, and reflux continued 5 minutes. The solution was cooled slowly to 0—5°C, granulated for 2 hours and the quinine salt of sorbinil recovered by filtration and dried at 40°C, 16.05 g, m.p. 113—122°C (dec), $[alpha]_D^{25} = -70.4°$. Step yield 77.9%.

In an optional step, the quinine salt of sorbinil (15.96 g) was recrystallized by dissolving in 79.8 ml isopropanol at reflux, concentrating to 44 ml, slow cooling to room temperature, diluting with 20 ml isopropanol, cooling to 0—5°C, granulating for 2 hours, filtering and drying at 40°C *in vacuo*, 14.38 g, m.p. 110.5—122°C (dec) $[alpha]_D^{25} = -71.0°$ (c = 1, methanol). Step yield: 90%.

Quinine salt of sorbinil (14.1 g, 25.1 mmole) was distributed between 350 ml ethyl acetate and 175 ml 1N HCl. The ethyl acetate layer was separated, washed 1 × 175 ml fresh 1N HCl, 1 × 175 ml $H_2O$ and 1 × 175 ml brine, dried ($MgSO_4$), evaporated to 25 ml *in vacuo*, chased 3 × 50 ml ethyl acetate to a final volume of 20 ml, cooled to 0—5°C, granulated 2 hours and sorbinil recovered by filtration, 4.72 g, m.p. 241—246°C, $[alpha]_D^{25} = +54.0°$ (c = 1, methanol). Step yield: 80%. Overall yield: 39.1%.

Example 5

Sorbinil via Its (+)-3-Aminomethylpinane and Quinine Salts

By substituting the same quantity of (+)-3-aminomethylpinane HCl for (−)-3-aminomethylpinane in the first stage of Example 1, the crude (+)-3-aminomethylpinane salt of the enantiomer of sorbinil is isolated in like quantity, having like melting point and like optical rotation, except of opposite sign. By the methods of Example 2, the combined mother liquor and wash, which contain a concentrate of the (+)-3-aminomethylpinane salt of sorbinil, is manipulated to yield a partially resolved concentrate of sorbinil, then converted to quinine salt of sorbinil and finally to sorbinil.

**Claims for the Contracting States: BE CH DE FR GB IT LI LU NL SE**

1. A process for preparing sorbinil, or a pharmaceutically acceptable cationic salt thereof, which consists in:

(a) crystallizing the (−)-3-aminomethylpinane salt of sorbinil from a solution of racemic hydantoin of the formula

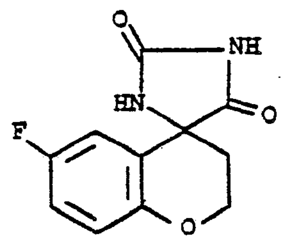

---(II)

in a solvent consisting of a lower alkanol or a mixture of lower alkanols; and

(b) converting said aminopinane salt to said sorbinil or pharmaceutically acceptable salt thereof.

2. A process for preparing sorbinil, or a pharmaceutically acceptable cationic salt thereof, which consists in:

(a) crystallizing either the (+)-3-aminomethylpinane or the (−)-2-amino-2-norpinane salt of the enantiomer of sorbinil from a solution of racemic hydantoin of the formula

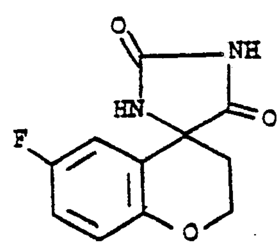

---(II)

in a solvent consisting of a lower alkanol or a mixture of lower alkanols;

(b) isolating a concentrate of sorbinil from the mother liquor thereof;

(c) crystallizing the quinine salt of sorbinil from a solution of the said sorbinil concentrate; and

(d) converting said quinine salt of sorbinil to said sorbinil or pharmaceutically acceptable salt.

3. A process according to claim 1 or claim 2, in which the solvent is a mixture of methanol and isopropanol in a ratio of about 1:1 by volume.

4. A chiral amine salt of sorbinil wherein said chiral amine is selected from the group consisting of (−)-3-aminomethylpinane, (+)-3-aminomethylpinane, (−)-2-amino-2-norpinane and quinine.

5. The chiral amine salt of claim 3 wherein the amine is (−)-3-aminomethylpinane.
6. The chiral amine salt of claim 3 wherein the amine is quinine.

**Claims for the Contracting State: AT**

1. A process for preparing sorbinil, or a pharmaceutically acceptable cationic salt thereof, which comprises the steps of:

(a) crystallizing the (−)-3-aminomethylpinane salt of sorbinil from a solution of racemic hydantoin of the formula

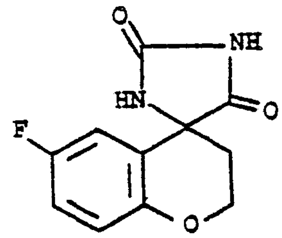

---(II)

in a solvent consisting of a lower alkanol or a mixture of lower alkanols; and

(b) converting said aminopinane salt to said sorbinil or pharmaceutically acceptable salt thereof.

2. A process for preparing sorbinil, or a pharmaceutically acceptable cationic salt thereof, which comprises the steps of:

(a) crystallizing either the (+)-3-aminomethylpinane or the (−)-2-amino-2-norpinane salt of the enantiomer of sorbinil from a solution of racemic hydantoin of the formula

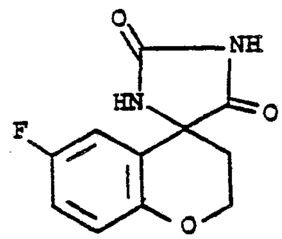

---(II)

in a solvent consisting of a lower alkanol or a mixture of lower alkanols;

(b) isolating a concentrate of sorbinil from the mother liquor thereof;

(c) crystallizing the quinine salt of sorbinil from a solution of the said sorbinil concentrate; and

(d) converting said quinine salt of sorbinil to said sorbinil or pharmaceutically acceptable salt.

3. A process according to claim 1 or claim 2, in which the solvent is a mixture of methanol and isopropanol in a ratio of about 1:1 by volume.

**Patentansprüche für die Vertragsstaaten: BE CH DE FR GB IT LI LU NL SE**

1. Verfahren zur Herstellung von Sorbinil oder einem seiner pharmazeutisch annehmbaren kationischen Salze, welches

(a) Kristallisieren des (−)-3-Aminomethylpinansalzes von Sorbinil aus einer Lösung von racemischem Hydantoin mit der Formel

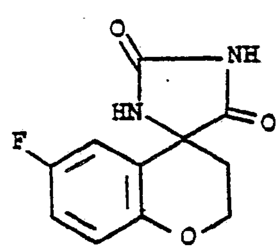

---(II)

in einem Solvens, das aus einem niederen Alkanol oder einer Mischung niederer Alkanole besteht, und

(b) Umwandeln des Aminopinansalzes zu Sorbinil oder einem seiner pharmazeutisch annehmbaren Salze umfaßt.

2. Verfahren zur Herstellung von Sorbinil oder einem seiner pharmazeutisch annehmbaren kationischen Salze, welches

(a) Kristallisieren entweder des (+)-3-Aminomethylpinan- oder (−)-2-Amino-2-norpinansalzes des Enantiomeren von Sorbinil aus einer Lösung von racemischem Hydantoin mit der Formel

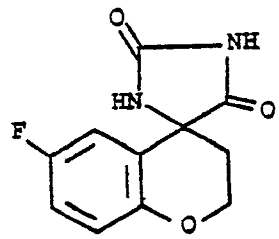

---(II)

in einem aus einem niederen Alkanol oder einer Mischung von niederen Alkanolen bestehenden Solvens,

(b) Isolieren eines Sorbinil-Konzentrats aus der Mutterlauge hiervon,

(c) Kristallisieren des Chininsalzes von Sorbinil aus einer Lösung dieses Sorbinil-Konzentrats und

(d) Umwandeln des Chininsalzes von Sorbinil in Sorbinil oder eines seiner pharmazeutisch annehmbaren Salze umfaßt.

3. Verfahren nach Anspruch 1 oder 2, in dem das Solvens eine Mischung aus Methanol und Isopropanol in einem Volumenverhältnis von etwa 1:1 ist.

4. Chirales Aminsalz von Sorbinil, wobei das chirale Amin aus der aus (−)-3-Aminomethylpinan, (+)-3-Amino-methylpinan, (−)-2-Amino-2-norpinan und Chinin bestehenden Gruppe ausgewählt ist.

5. Chirales Aminsalz nach Anspruch 3, worin das Amin (−)-3-Aminomethylpinan ist.

6. Chirales Aminsalz nach Anspruch 3, worin das Amin Chinin ist.

**Patentansprüche fur den Vertragsstaat: AT**

1. Verfahren zUur Herstellung von Sorbinil oder einem seiner pharmazeutisch annehmbaren kationischen Salze, welches die Schritte:

(a) Kristallisieren des (−)-3-Aminomethylpinansalzes von Sorbinil aus einer Lösung von racemischem Hydantoin mit der Formel

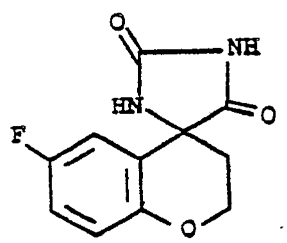

---(II)

in einem Solvens, das aus einem niederen Alkanol oder einer Mischung niederer Alkanole besteht, und

(b) Umwandeln des Aminopinansalzes zu Sorbinil oder einem seiner pharmazeutisch annehmbaren Salze umfaßt.

2. Verfahren zur Herstellung von Sorbinil oder einem seiner pharmazeutisch annehmbaren kationischen Salze, welches die Schritte:

(a) Kristallisieren entweder des (+)-3-Aminomethylpinan- oder (−)-2-Amino-2-norpinansalzes des Enantiomeren von Sorbinil aus einer Lösung von racemischem Hydantoin mit der Formel

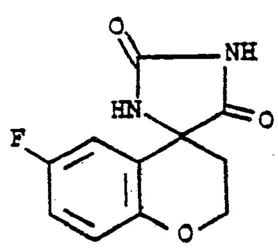

---(II)

in einem aus einem niederen Alkanol oder einer Mischung von niederen Alkanolen bestehenden Solvens,

(b) Isolieren eines Sorbinil-Konzentrats aus der Mutterlauge hiervon,

(c) Kristallisieren des Chininsalzes von Sorbinil aus einer Lösung dieses Sorbinil-Konzentrats und

(d) Umwandeln des Chininsalzes von Sorbinil in Sorbinil oder eines seiner pharmazeutisch annehmbaren Salze umfaßt.

3. Verfahren nach Anspruch 1 oder 2, in dem das Solvens eine Mischung aus Methanol und Isopropanol in einem Volumenverhältnis von etwa 1:1 ist.

**Revendications pour les Etats contractants: BE CH DE FR GB IT LI LU NL SE**

1. Procédé de préparation du sorbinil ou d'un sel cationique pharmaceutiquement acceptable de ce composé, qui comprend:

(a) la cristallisation du sel de (−)-3-aminométhylpinane du sorbinil dans une solution d'hydantoïne racémique de formule

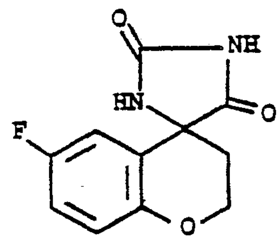

---(II)

dans un solvant consistant en un alcanol inférieur ou en un mélange d'alcanols inférieurs; et

(b) la transformation dudit sel d'aminopinane en ledit sorbinil ou son sel pharmaceutiquement acceptable.

2. Procédé de préparation du sorbinil ou d'un sel cationique pharmaceutiquement acceptable de ce composé, qui comprend:

(a) la cristallisation du sel de (+)-3-aminométhylpinane ou de (−)-2-amino-2-norpinane de l'énantiomère du sorbinil dans une solution d'hydantoïne racémique de formule

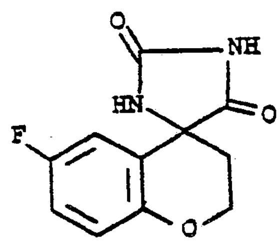

---(II)

dans un solvant consistant en un alcanol inférieur ou un mélange d'alcanols inférieurs;

(b) l'isolement d'un concentré de sorbinil de sa liqueur-mère;

(c) la cristallisation du sel quinine du sorbinil dans une solution dudit concentré de sorbinil; et

(d) la transformation dudit sel de quinine du sorbinil en ledit sorbinil ou son sel pharmaceutiquement acceptable.

3. Procédé suivant la revendication 1 ou la revendication 2, dans lequel le solvant est un mélange de méthanol et d'isopropanol dans un rapport en volume d'environ 1:1.

4. Sel d'amine chirale du sorbinil, dans lequel ladite amine chirale est choisie dans le groupe comprenant le (−)-3-aminométhylpinane, le (+)-3-aminométhylpinane, le (−)-2-amino-2-norpinane et la quinine.

5. Le sel d'amine chirale suivant la revendication 3, dans lequel l'amine est le (−)-3-aminométhylpinane.

6. Sel d'amine chirale suivant la revendication 3, dans lequel l'amine est la quinine.

**Revendications pour l'Etat contractant: AT**

1. Procédé de préparation du sorbinil ou d'un sel cationique pharmaceutiquement acceptable de ce composé, qui comprend comme étapes:

(a) la cristallisation du sel de (−)-3-aminométhylpinane du sorbinil dans une solution d'hydantoïne racémique de formule

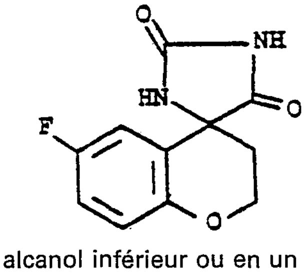

---(II)

dans un solvant consistant en un alcanol inférieur ou en un mélange d'alcanols inférieurs; et

(b) la transformation dudit sel d'aminopinane en ledit sorbinil oiu son sel pharmaceutiquement acceptable.

2. Procédé de préparation du sorbinil ou d'un sel cationique pharmaceutiquement acceptable de ce composé, qui comprend comme étapes:

(a) la cristallisation du sel de (+)-3-aminométhylpinane ou de (−)-2-amino-2-norpinane de l'énantiomère du sorbinil dans une solution d'hydantoïne racémique de formule

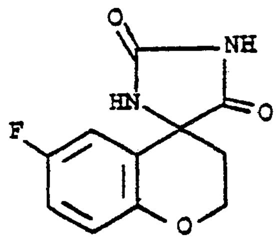

---(II)

dans un solvant consistant en un alcanol inférieur ou un mélange d'alcanols inférieurs;

(b) l'isolement d'un concentré de sorbinil de sa liqueur-mère;

(c) la cristallisation du sel quinine du sorbinil dans une solution dudit concentré de sorbinil; et

(d) la transformation dudit sel de quinine du sorbinil en ledit sorbinil ou son sel pharmaceutiquement acceptable.

3. Procédé suivant la revendication 1 ou la revendication 2, dans lequel le solvant est un mélange de méthanol et d'isopropanol dans un rapport en volume d'environ 1:1.